# EUROPEAN PATENT APPLICATION

(11) **EP 4 548 926 A1**
(43) Date of publication of application: **07.05.2025**
(21) Application number: 22949504.9
(22) Date of filing: 09.11.2022
(51) Int. Cl.: A61K 38/16, A61P 25/28, A61P 43/00, C07K 14/46, C12N 15/12

(54) **Y-SECRETASE INHIBITOR, AB-PEPTIDE PRODUCTION INHIBITOR, AND PHARMACEUTICAL**

(30) Priority: 30.06.2022 JP 2022106682
(71) Applicant: Tohoku University, Sendai-shi, Miyagi 980-8577 (JP)
(72) Inventor: FUTAI, Eugene, Sendai-shi, Miyagi 980-8577 (JP); SAKAKIBARA, Rina, Sendai-shi, Miyagi 980-8577 (JP); SEKI, Masayoshi, Sendai-shi, Miyagi 980-8577 (JP); HIDAKA, Masafumi, Sendai-shi, Miyagi 980-8577 (JP); OGAWA, Tomohisa, Sendai-shi, Miyagi 980-8577 (JP)
(74) Representative: Winter, Brandl - Partnerschaft mbB
(86) International application number: PCT/JP2022/041795
(87) International publication number: WO 2024/004231

(57) **Abstract**

An object to be achieved by the present invention is to provide a novel γ-secretase inhibitor including, as an active ingredient, a substance whose γ-secretase inhibitory activity has heretofore been unknown. The present invention provides a γ-secretase inhibitor including a three-finger toxin.

## Description

### Technical Field

### [Cross-reference to Related Application]

This application claims priority from Japanese Patent Application No. 2022-106682, filed on June 30, 2022, the entire disclosure of which is incorporated herein by reference. The present invention relates to a γ-secretase inhibitor, an Aβ-peptide production inhibitor, and a pharmaceutical.

### Background Art

Dementia reduces the cognitive function of a brain to cause troubles in the entirety of a daily life. In Japan where aging is significant, the number of the patients of the dementia has been increasing, and it is said that the number is estimated to reach about 7,000,000 in 2025. In addition, Alzheimer's disease (AD) accounts for 70% of the cases of the dementia, and the accumulation of an amyloid β peptide (Aβ) (senile plaque) has been considered to be a cause therefor. The Aβ is produced as follows: the extracellular domain of an amyloid precursor protein (APP) is cleaved by a β-secretase; and then, a region (APPC99) remaining in a membrane is cleaved by a γ-secretase that is an intramembrane-cleaving protease. The cleavage by the γ-secretase is stepwise, and as a result of endopeptidase-like cleavage (ε-cleavage) and trimming by carboxypeptidase-like cleavage (γ-cleavage) for each of 3 or 4 amino acid residues at a carboxy terminal subsequent thereto, an Aβ40 or an Aβ43 is produced from an Aβ49, and an Aβ42 or an Aβ38 is produced from an Aβ48. Although many of the peptides to be produced are the Aβ38 and the Aβ40, the Aβ42 and the Aβ43 each have high aggregability, and hence have been recognized as toxic molecules.

γ-Secretase targeting drugs intended for the treatment of AD are mainly classified into a γ-secretase inhibitor (GSI) and a γ-secretase modifier (GSM), and a low-molecular weight compound has been developed. Although semagacestat and avagacestat each serving as the GSI were subjected to trials up to phase III clinical trials, and were expected as drugs specifically inhibiting the cleavage of the APP, the clinical trials were stopped because serious side effects and the deterioration of a cognitive function were observed. A subsequent verification experiment has shown that semagacestat is a pseudo-inhibitor that does not inhibit Aβ production and inhibits the discharge of a decomposition product to the outside of a cell. The GSI has such many points to be overcome as described below: the cleavage of the APP can be specifically inhibited without inhibition of the cleavage of Notch; and the cytotoxicity of an APPC99 fragment accumulated by the GSI is removed. The GSM has been expected to reduce the production ratio of the Aβ42 or the Aβ43 through an increase in trimming activity by the stabilization of a transition state complex of an enzyme and a substrate. In addition to nonsteroidal anti-inflammatory drugs (NSAIDs) that are each the first GSM, a second-generation GSM (an acidic GSM, GSM-1) and a third-generation GSM (a phenylimidazole-type GSM, E2012) have been developed from cell screening. Although some of the GSMs have been subjected to clinical trials, the trials have been stopped. Meanwhile, in 2019, a human gene recombinant monoclonal antibody aducanumab serving as an immunotherapeutic drug for the Aβ passed a phase III clinical trial, and in 2021, aducanumab was rapidly approved by the United States Food and Drug Administration (FDA). The passage and the approval have made a giant step toward the radical treatment of AD for which no effective therapeutic drug is available. However, further development of a dementia therapeutic drug has been strongly desired.

### Citation List

### Patent Literature

PTL 1: WO 2020/040106 A1

### Non-patent Literature

NPL 1: Methods. Mol. Biol. 2049, 403-417 (2019)
NPL 2: J. Biol. Chem. 284, 13013-13022 (2009)
NPL 3: Biochem. Biophys. Res. Commun. 377, 141-145
(2008) NPL 4: Sci. Rep. 8, 10.1038/s41598-018-28749-4 (2018).
NPL 5: Toxicon, 56 (6), 855-867 (2010).

### Summary of Invention

### Technical Problem

An object to be achieved by the present invention is to provide a novel γ-secretase inhibitor including, as an active ingredient, a substance whose γ-secretase inhibitory activity has heretofore been unknown.

### Solution to Problem

Under such circumstances, the inventors of the present invention have made extensive investigations, and as a result, have found that a three-finger toxin peptide inhibits the activity of the decomposition of an amyloid precursor protein (APP) by a γ-secretase. The present invention is based on such novel finding.

Accordingly, the present invention provides the following items:
Item 1. A γ-secretase inhibitor including a three-finger toxin.
Item 2. An Aβ-peptide production inhibiitor including a three-finger toxin.
Item 3. A pharmaceutical for preventing or treating dementia including a three-finger toxin.
Item 4. The γ-secretase inhibitor according to Item 1, the Aβ-peptide production inhibiitor according to Item 2, or the pharmaceutical according to Item 3, wherein the three-finger toxin is derived from *Protobothrops flavoviridis.*
Item 5. The γ-secretase inhibitor according to Item 1, the Aβ-peptide production inhibiitor according to Item 2, or the pharmaceutical according to Item 3, wherein the three-finger toxin is formed of:
   a polypeptide including an amino acid sequence set forth in SEQ ID NO: 1 or an amino acid sequence obtained by substituting, deleting, or adding one or a plurality of amino acids in the amino acid sequence set forth in SEQ ID NO: 1;
   a polypeptide including an amino acid sequence set forth in SEQ ID NO: 2 or an amino acid sequence obtained by substituting, deleting, or adding one or a plurality of amino acids in the amino acid sequence set forth in SEQ ID NO: 2;
   a polypeptide including an amino acid sequence set forth in SEQ ID NO: 3 or an amino acid sequence obtained by substituting, deleting, or adding one or a plurality of amino acids in the amino acid sequence set forth in SEQ ID NO: 3; or
   a polypeptide including an amino acid sequence set forth in SEQ ID NO: 4 or an amino acid sequence obtained by substituting, deleting, or adding one or a plurality of amino acids in the amino acid sequence set forth in SEQ ID NO: 4:
      SEQ ID NO: 1: 
MKTLLLILGVVAFVYLEPGYTRKCCIYPKSALFCREVQICPADEGMCFKMWNSTDRLMERTYYVRG
CAAACPNPVGDEEVYCCVTDNCNK.
      SEQ ID NO: 2: MKTLLLILGVVAFVYLDSGFSLICNTCNRTVRIPLIPCDPVTCPEGLDQCYANRTGILRMKVERG CTTNCTQTLTLMCCKRDMCN
      SEQ ID NO: 3: 
MKTHLLALVMVAFLYLEPGYTLRCHSCVGYVCKEFEFCKPEQKQCIYLINRKKYSGCSRNCPSAG
ANELVKCCATDRCNLEIFCW
      SEQ ID NO: 4:

MKTLLLILGVVAFVYLDSGFSLKCEQCNLPNCDFLPDVRCPKGLDQCYKKWNETAKVFERGCTAN
CNEDEQTRCCKRNGCNF

### Advantageous Effects of Invention

According to the present invention, the novel γ-secretase inhibitor including, as an active ingredient, a substance whose γ-secretase inhibitory activity has heretofore been unknown can be provided. The three-finger toxin serving as the active ingredient of the present invention can be expected to have higher specificity (be reduced in side effect) because the toxin is a medium-molecular weight inhibitor, such as a protein or a peptide.

### Brief Description of Drawings

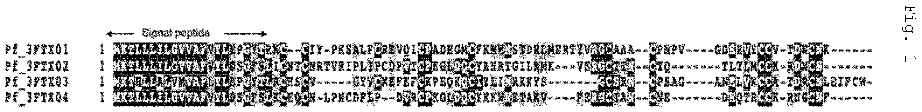

Fig. 1 is an illustration of the amino acid sequences of *Protobothrops flavoviridis* three-finger toxins Pf3FTX01 to Pf3FTX04.
Fig. 2 to Figs. 4 are photographs and graphs showing the inhibition of the cleavage of an amyloid precursor protein. Fig. 2 is a set of photographs showing the interaction/inhibition evaluation of a *Protobothrops flavoviridis* 3FTX by the yeast growth evaluation of a γ-secretase-expressing yeast.
Fig. 3 is a graph showing the interaction/inhibition evaluation of the *Protobothrops flavoviridis* 3FTX by the β-galactosidase assay of the γ-secretase-expressing yeast.
Figs. 4 are a photograph and graphs showing the evaluation of the γ-secretase inhibition ability of the *Protobothrops flavoviridis* 3FTX by an in vitro Aβ production assay. Fig. 4(A) is a photograph showing western blot analysis with an anti-Aβ antibody. Fig. 4(B) is a set of graphs showing determination with the signal intensities of the respective bands (an Aβ, a C55, and a C55 dimer).
Fig. 5 and Fig. 6 are photographs and a graph showing the inhibition of the cleavage of Notch. Fig. 5 is a set of photographs showing the interaction/inhibition evaluation of the *Protobothrops flavoviridis* 3FTX by the yeast growth evaluation of the γ-secretase-expressing yeast.
Fig. 6 is a graph showing the interaction/inhibition evaluation of the *Protobothrops flavoviridis* 3FTX by the β-galactosidase assay of the γ-secretase-expressing yeast.
Figs. 7 are each a graph showing the interaction/inhibition evaluation of a related-art GSI by the β-galactosidase assay of a γ-secretase-expressing yeast.

### Description of Embodiments

In the present invention, the terms "protein" and "peptide" are each used in a meaning including an oligopeptide and a polypeptide. In addition, herein, the terms "protein" and "peptide" each encompass both of a protein modified with a glycan or the like and an unmodified protein, unless otherwise stated. The same applies to a protein that is not specified to be a protein.

### Pharmaceutical

In one embodiment, the present invention provides a pharmaceutical for preventing or treating dementia including a three-finger toxin. The three-finger toxin is a peptide having a characteristic structure called a three-finger structure (TFS) formed of (typically 4 or 5) disulfide bonds. In the present invention, the three-finger toxin is, for example, a toxin derived from a snake. Examples of the snake include *Protobothrops flavoviridis, Protobothrops elegans, Protobothrops mucrosquamatus, Protobothrops tokarensis,* a rattlesnake, an adder, a brown snake, and a brown tree snake. In addition, examples of the three-finger toxin include the following toxins:
Toxin type
Mambalgin type
   NXSH_BUNMU_gi|1171877|sp|P43445.1, Mambalgin-1_gi|425936479|sp|P0DKR6.1|, NXS6_OPHHA_gi|123907651|sp|Q2VBP2.1|, NXS14_OPHHA_gi|123910515|sp|Q2VBP0.1|, SNTX26_gi|123910514|sp|Q2VBN9.1, NXSA2_MICLL_gi|704043470|sp|K9MCX0.1|, NXS1_MICCO_gi|519882254|sp|C6JUP1.1|, pt&#65343_alpha&#12540_neuroto, NXS7_PSETE_gi|24638074|sp|Q9W7J6.1|, 3FTx-Oxy5_gi|166200542|sp|A7X4S7.1, Scutelatoxin_gi|82227128|sp|Q4VRI0.1|, NXS3_0XYSC_gi|254772681|sp|A8HDK2.1|, neurotoxin513V5_gi|334854586|gb|AEH05952.1|, 3FTx-Oxy6_gi|166200544|, Oo_3FTx-Oxy6_A7X4T2.1, 3FTx-Aze-1_gi|478415062|, Af_3FTx-Aze-1_AGI97280.1, Sce_3FTX2_gi|109255014|, Sce_3FTx2_ABG27005.1, Sce_3FTX2pre_gi|167614283|, Sce_3FTx2_ABZ89717.1, Pm_3FTx-3-like_XP_015678603.1, habu1_s4579_g14475_Pfsv3FTX04, Pm_mambalgin-1-like_XP_015683100.1, Sce_3FTX4B_gi|109255020|, Sce_3FTx4B_ABG27008.1, Sce_3FTX4A_gi|109255018|, Sce_3FTx4A_ABG27007.1, Sce_3FTx4_ABZ89718.1, Sce_3FTX4pre_gi|167614285|, Sce_3FTX5pre_gi|1676142871| Sce_3ETx5_ABZ89719.1, Sce_3FTX1gi|109255012|, Sce_3FTx1_ABG27004.1, habu1_s4579_g14477_Pfsv3FTX02
Non-conventional
   Mc_3FTx_gi|241995609|gb|ACS74999.1|, Mt_3FTx18_JAS05203.1, NXAH8_MICCO_gi|17368232|sp|P58370.1, non-conventional3FTX-4_gi|294961056|gb|ADF50019.1|, Bf_non-conventional3FTX-2_gi|294961052|gb|ADF50017.1|, Bf_non-conventional3FTX-1_gi|294961050|gb|ADF50016.1|, NXLH2_BUNMU_gi|25453174|sp|Q9YGH9.1|, NXL4_MICFR_gi|298351749|sp|P86423.1|, NXLR1_BUNMU_gi|128949|sp|P15817.1|, NXL6_BUNMU_gi|14194996|sp|Q9W729.1|, LsIII_gi|32140571|dbj |BAC78204.1|
Alpha-elapitoxin (long a-neurotoxin/nicotinic-AchRs) Alpha-EPTX-Dv2b_gi_|128926|sp|P01394.1|, Alpha-elapitoxin-Dv2b, Alpha-EPTX-Dv2a_gi|128941|sp|P01395.11, Alpha-elapitoxin-Dv2a, Alpha-EPTX-Djk2a_gi|128924Isplp01393.11| Alpha-elapitoxin-Djk2a, Alpha-EPTX-Dpp2c_gi|128940|sp|p01397.21, Alpha-elapitoxin-Dpp2c, Alpha-EPTX-Dpp2b_giI128946Ispl^{_}25667.11, Alpha-elapitoxin-Dpp2b, Alpha-EPTX-Dpp2a_giI128925Ispl^{p}01396.11, Alpha-elapitoxin-Dpp2a, Alpha-elapitoxin-dpp2d, NXL28O0PHHA_gi|123916246|sp|IQ2VBP4.11, Alpha-EPTX-Oh2b_gi.|l 66214959|sp|P82662.2|, NXL₃₇_OPHHAg2193162|sp|Q53B59.1|, Bm_3FT_em B692.1
Muscarinic2
   Bf_short-3FTX6_gi|294961046|gb|ADF50014.1|, Bf_short-3FTX2 gi|294961038|gb|ADF50010.1|, Bf_short-3FTX3_gi|294961040|gb|ADF500111|, TML3H_OPHHA_gi|172048118|sp|A8N28611, TXML_BUNMU_gi|12230781|sp|Q9W727.1|, ToxinS5C1_gi|136518|sp|P01413 .1|, Toxin_S5C1, Muscarinic, Muscarinic-m2-toxin, NXS1_DENPO_gi|128961|sp|P01416.1|, Short_neurotoxin1, Toxin-S5C10_gi|136517|sp|P01419.1|, S5C10, Toxin-FS-2_gill36536|sp|P01414.11, Toxin-FS-2, ToxinC13S1C1_gi|136513|sp|P18329.1|, C13S1C1, Toxin-4.9.6_gi|136515|sp|P01405.1|, Toxin-4.9.6, ToxinS5C4_gi|136519|sp|P01406.1|, Toxin_S5C4, Toxin-DaF8_gi|464972|sp|P01404.2|, Toxin TA2
Fasciclin (Acetylcholinesterase inhibitor)
   TXAC_DENPO_gi|136533|sp|P25681.1|, Acetylcholinesterase-toxinC, Fasciculin-1 gi|116256102|sp|P0C1Y9.1, Fasciculin-1, Fasciculin-Ti_gi|1421398|pdb|1FSS|, TXFA2_DENAN, Fasciculin-2
Short-weaktoxin
   NXS58_MICAT_gi|519882267|sp|F5CPD9.1|, NXS57_MICAT_gi|519882266|sp|F5CPD8.1|, Pr_3FTX_gi|526252745|dbj|BAN63794.1|, Mi_short-neurotoxin_gi|630870092|gb|AHZ08818.1|, Weak_toxin_DE-1_gi|182705233|sp|P01412.2|, Oh_Weak-toxin_DE-1_gi|82199672|, Oh_Weak
Cardiotoxin
   Nn_Cardiotoxin, Na_Cytotoxin5L_gi|146395925|, Nn_Cytotoxin, Na_CytotoxinA5_gi|51316994|, CX7P_NAJAT_gi|2500805|sp|Q91996.1|, CX7A_NAJAT_gi|2500804|sp|Q91126.1|, CXH5_NAJAT_gi|46396045|sp|Q9W716.1|, NA_CTX7_gi|1345873|sp|P49122.1], cardiotoxinN_gi|1000509|emb|CAA90966.1|, CTX6_NAJAT_gi|41688512|sp|Q98965.1|, CTX2A_NAJSPgi|41688523|sp|Q9PST4.1|, CTX2B_NAJSP_gi|41688522|sp|Q9PST3.1|, CTX4B_NAJSP_gi|41688442|sp|O73856.2|, CTX4A_NAJSP_gi|41688448|sp|O93473.1|, Na_CTX-3_gi|4753827|emb|CAB42055.1|, Naja_CTX-3_gi|41688806|sp|P60301.1|, CX3A_NAJAT_gi|2500800|sp|Q98959.1|, CTX1_NAJSP_gi|41688446|sp|O93471.1|, CX3B_NAJAT_gi|18281731|sp|Q98960.2|, Naja_CTX2_gi|41688513|sp|Q9DGH9.1|, CTXA2_NAJAT_gi|2507397|sp|P01442.2|, CX5_NAJAT_gi|2500802|sp|Q98961.1|, CTXA1_NAJAT_gi|41688809|sp|P60304.1|, Na_cardiotoxin-1_gi|4753829|emb|CAB42056.1|, Nn_cardiotoxin-1_gi|1000502|emb|CAA90962.1|, CX16_NAJAT_gi|41688507|sp|Q91135.1|, CX1C_NAJAT_gi|41688455|sp|P79810.1|, CX1D_NAJAT_gi|2500799|sp|Q98958.1|, CX1A_NAJAT_gi|2500798|sp|Q98957.1|, CX1B_NAJAT_gi|2500797|sp|Q98956.1|, CTX8_NAJAT_gi|41688506|sp|Q91124.1|, CX3D_NAJAT_gi|2500803|sp|Q98962.1|, Na_3FTX_gi|307602767|gb|ADN67578.1|
Weaktoxin
   NTX2_NAJAT_gi|46396552|sp|Q9W717.1|, Bc_Weak_toxin1_Q8AY51.1, NXW1_BUNCA_gi|82212020|sp|Q8AY51.1|, Bc_Weaktoxin_Q8AY50.1, NXW2_BUNCA_gi|82212019|sp|Q8AY50.1|, Bf_nc3FTx-7_ADF50022.1, Bf_nc3FTx-6_ADF50021.1, Bf_nc_3FTx-5_ADF50020.1, NAJAT_NNAM1_Q9YGI2.1, Nn_CAA04578.1, Nn_un_emb|CAA04578.1, Nn_NNAM2I_P60814.1
Muscarinic (muscarinic-AchRs)
   Muscarinic-toxin2, TXM2_DENAN_gi|136545|sp|P18328.1|, Muscarinic-m1-toxin1, NXM11_DENAN_gi|31077032|sp|Q8QGR0.1|, Muscarinic-alpha, TXMA_DENPO_gi|1351327|sp|P80494.1| MTx1, TXM1_DENAN_gi|2829716|sp|P81030.1|, MT4, TXM4_DENAN_gi|46397052|sp|Q9PSN1.1|, Rho-EPTX-Dalb, Rho-EPTX-Da1b_gi|306756326|sp|P86419.1|, MT-beta, TXMB_DENPO_gi|1351328|sp|P80495.1|, Synergistic-likeCM-3, Muscarinic-CM-3_gi|136461|sp|P25518.1|, Rho-EPTX-Dala, Rho-EPTX-Da1a_gi|263519260|sp|P85092.1|, MT3, TXM3_DENAN_gi|42560546|sp|P81031.2|
Synergistic
   DaSynTox, Synergistic-type_C9S3, Synergistic-C9S3-1_gi|136439|sp|P01408.1|, Synergistic-like_venom_protein, Synergistic-like_gi|136460|splP17696.1|, Synergistic-type_C8S2, Synergistic-C8S2-2 gi|136438|sp|P01411.1|, Synergistic-type_C8S2_, Synergistic-C8S2-1_gi|136437|sp|P01410.1|, Synergistic-type_S2C4, Synergistic-S2C4_gi|136436|sp|P01407.1|
Pf3FTX01 type (new function type)
   Pf_3FTx_BAN89397.1, Pf_3FTX_gi|547223049|, habu1_s4579_g14476_Pfsv3FTX01, Pm_3FTx-like_XP_015678608.1, A5X2W8.1_3SX3_SISCA, TX3_SISCA_gi|166229885|, 3FTx-Psa1_A7X3M3.1, 3FTx-Psa1_gi|166229884|, 3FTx-Lei1_A7X3R6.1, 3FTx-Lei1_gi|166229883|sp|A7X3R6.1|, Tb_3FTx-Tri1_ABU68475.1, 3FTx-Tri1_gi|156485228|, Tj_3FTx-Thr5_A7X6I4.1, 3FTx-Thr5_gi|166200543|sp|A7X6I4.1|, Dt_3FTx-Dis4_A7X6J0.1, 3FTx-Dis4_gi|166229886|sp|A7X6J0.1|, Ap_3FTx1_ANN23934.1
Ancestor type
   habu1_s4579_g14478_Pfsv3FTX03, 20140925_all_comp511930_c0_seq, Bi_3FTz-6a_JAS04622.1, Bi_3FTx-6c_JAS04620.1, Bi_3FTx6a_JAS04622.1, Bi_3FTx-6b_JAS04621.1, Bi_3FTx-5_JAS04623.1, Td_3FTx-Tell ABU68471.1, 3FTx-Tel1gi|156485220|, Dt_3FTx-Dis1_ABU68474.1, 3FTx-Dis1_gi|156485226|gb|ABU68474.1|, Pm_3FTx_JAS03139.1, Pm_3FTx_JAS03140.1, 3FTx-Lio3_gi|156485224|gb|ABU68473.1|, 3FTx-Lio2,
Non-toxin type
CD59 glycoprotein
   Pb_CD59_gi|602655005|ref|XP_007433279.1|, Ss_CD59_gi|5106773|gb|AAD39837.1|AF058328_1, Ch_CD59_gi|548496373|ref|XP_005690130.1|, Oa_CD59_gi|426245276|ref|XP_004016439.1|, Bt_CD59_gi|82697317|ref|NP_001032523.1|, SSHCSHL338_gi|675304909|gb|AIL82450.1|, Bm_CD59_gi|555957639|ref|XP_005890947.1|, Sp_sperm-acrosome-membrane-associated-protein 4-like_gi|657572316|ref|XP_008289768.1|, gi|2209360|gb|AAC17925.1|, Ci_neurotoxin/C59/Ly-6-like_ gi|38456062|gb|AAR20998.1|, Dr_CD59_gi|288856246|ref|NP_001165779.1|, Ca_CD59_gi|514483125|gb|AGO58848.1|, Dr_CD59_gi|326665256|ref|XP_003198001.1|, Dr_CD59_gi|688539541|ref|XP_009297186.1|, Lc_CD59B_gi|557006867|ref|XP_006004860.1|, Ps_CD59A_gi|558127352|ref|XP_006115537.1|, Cpb_CD59A_gi|530640612|ref|XP_005307066.1|, Cm_CD59A_gi|591364151|ref|XP_007056977.1|, Lm_3FTX_gi|88861958|gb|ABD52883.1|, 20140925_all_comp593999_c3_seq26(-), Pf3FTX-5a, Pf3FTX-5b, Pf3FTX-5d, Pf3FTX-5c_3FTX-5_, Pf3FTX-5f, Pf3FTX-5e, plethodontid-modulating-factor, 20140925_all_comp512785_c1_seq2, 20140925_all_comp512785_c1_seq3, Pf3FTX-6, Ld_CD59_gi|700411410|ref|XP_009959781.1|, Mu_urokinase_plasminogen_activator_surface_receptor-like_gi|527273543|ref|XP_005154552.1|, Mv_urokinase_plasminogen_activator_surface_receptor-like_gi|675425919|ref|XP_008926741.1|, Cs_CD59_gi|657786850|ref|XP_008320441.1|, Lo_ly-6D_gi|573874327|ref|XP_006625562.1|
Ly6
   Jj_ly6D-like_gi|507544770|ref|XP_004656355.1|, Md_ly-6_gi|334325844|ref|XP_001381791.2|, Oa_ly-6_gi|620963972|ref|XP_001512986.2|, Tml_prostate-stem-cell-antigen_gi|471411749|ref|XP_004387427.1|, Cj_prostate-stem-cell-antigen_gi|296227136|ref|XP_002759243.1|, Pta_prostate_stem_cell_antigen_gi|591293222|ref|XP_007073897.1|, Gg_Ly-6E_gi|45382129|ref|NP_990106.1|, Fg_ly6E-like_gi|678977961|gb|KFV90977.1|, Oh_ly6E-like_gi|700388949|ref|XP_009934707.1|, Mu_ly-6E_gi|704574650|ref|XP_010186557.1|, Ee_ly-6_gi|617554744|ref|XP_007535106.1|, Mm_Ly-6/uPAR_gi|388453705|ref|NP_001253551.1|, Cs_ly-6X2_gi|635050497|ref|XP_007999891.1|, Op_ly-6_gi|504136695|ref|XP_004580813.1|.

Of the three-finger toxins, toxins classified into, for example, the Pf3FTX01 type (new function type), the mambalgin type, and the ancestor type are preferred.

Although the size of the three-finger toxin is not limited, a toxin having, for example, 40 to 130 amino acids, preferably 50 to 116 amino acids, more preferably 60 to 88 amino acids is available.

In a preferred embodiment, the three-finger toxin is, for example, a toxin having an amino acid sequence set forth in the following general formula:

MKTR¹LLR²LR³R⁴VAFR⁵YLR⁶R⁷GR⁸R⁹R¹⁰ (R¹¹)ₙ₁C(R¹²)ₙ₂^{C}(R¹³)ₙ₃C(R¹⁴)ₙ₄C(R¹⁵)ₙ₅P(R¹⁶ ) ₙ₆R¹⁷(R¹⁸)ₙ₇C(R¹⁹)ₙ₈R²⁰(R²¹)ₙ₉(R²²)ₙ₁₀GC(R²³)ₙ₁₁R²⁴C(R²⁵)ₙ₁₂R²⁶(R²⁷)ₙ₁₃CC(R²⁸)ₙ₁₄ R²⁹R³⁰(R³¹)ₙ₁₅CN(R³²)ₙ₁₆

where R¹ represents L or H, preferably L,
R² represents I or A, preferably I,
R³ represents G or V, preferably G,
R⁴ represents V or M, preferably V,
R⁵ represents V or L, preferably V,
R⁶ represents E or D,
R⁷ represents P or S,
R⁸ represents Y or F,
R⁹ represents T or S,
R¹⁰ represents R or L,
R¹¹ represents A, C, D, E, F, G, H, I, K, L, M, N, P, Q, R, S, T, V, W, or Y, preferably I, K, or R,
n1 represents an integer of from 0 to 2, preferably 1,
R¹² represents A, C, D, E, F, G, H, I, K, L, M, N, P, Q, R, S, T, V, W, or Y, preferably E, H, N, Q, S, or T,
n2 represents an integer of from 0 to 4, preferably from 0 to 2, R¹³ represents A, C, D, E, F, G, H, I, K, L, M, N, P, Q, R, S, T, V, W, or Y, preferably A, I, F, G, K, L, N, P, R, S, T, V, or Y, n3 represents an integer of from 3 to 11, preferably from 4 to 10,
R¹⁴ represents A, C, D, E, F, G, H, I, K, L, M, N, P, Q, R, S, T, V, W, or Y, preferably D, E, F, I, K, L, P, Q, R, T, or V, more preferably D, E, I, L, P, Q, R, T, or V,
n4 represents an integer of from 3 to 8, preferably from 4 to 7, R¹⁵ represents A, C, D, E, F, G, H, I, K, L, M, N, P, Q, R, S, T, V, W, or Y, preferably K,
n5 represents an integer of from 0 to 2, preferably 0 or 1,
R¹⁶ represents A, C, D, E, F, G, H, I, K, L, M, N, P, Q, R, S, T, V, W, or Y, preferably A, D, E, G, K, L, or Q,
n6 represents an integer of from 2 to 5, preferably 3 or 4,
R¹⁷ represents G or Q,
R¹⁸ represents A, C, D, E, F, G, H, I, K, L, M, N, P, Q, R, S, T, V, W, or Y, preferably M,
n7 represents an integer of from 0 to 2, preferably 0 or 1,
R¹⁹ represents A, C, D, E, F, G, H, I, K, L, M, N, P, Q, R, S, T, V, W, or Y, preferably A, F, I, K, L, M, N, R, W, or Y, more preferably A, F, K, M, N, R, W, or Y,
n8 represents an integer of from 3 to 5, preferably 4,
R²⁰ represents N or T,
R²¹ represents A, C, D, E, F, G, H, I, K, L, M, N, P, Q, R, S, T, V, W, or Y, preferably A, D, E, F, G, I, K, L, M, R, S, T, V, or Y,
n9 represents an integer of from 4 to 12, preferably from 5 to 11,
R²² represents R,
n10 represents an integer of from 0 to 2, preferably 0 or 1, more preferably 1,
R²³ represents A, C, D, E, F, G, H, I, K, L, M, N, P, Q, R, S, T, V, W, or Y, preferably A, S, T, or R, more preferably A, T, or R, n11 represents an integer of from 0 to 3, preferably 1 or 2,
R²⁴ represents A or N,
R²⁵ represents A, C, D, E, F, G, H, I, K, L, M, N, P, Q, R, S, T, V, W, or Y, preferably A, D, E, G, P, N, Q, S, T, or V, more preferably D, E, G, P, N, Q, T, or V,
n12 represents an integer of from 2 to 7, preferably from 3 to 6, R²⁶ represents E or L,
R²⁷ represents A, C, D, E, F, G, H, I, K, L, M, N, P, Q, R, S, T, V, W, or Y, preferably E, K, L, M, Q, R, T, V, or Y, more preferably E, L, M, Q, R, T, V, or Y,
n13 represents an integer of from 2 to 4, preferably 3,
R²⁸ represents A, C, D, E, F, G, H, I, K, L, M, N, P, Q, R, S, T, V, W, or Y, preferably A, K, or V, more preferably K or V,
n14 represents an integer of from 0 to 2, preferably 1,
R²⁹ represents T or R,
R³⁰ represents D or N,
R³¹ represents A, C, D, E, F, G, H, I, K, L, M, N, P, Q, R, S, T, V, W, or Y, preferably G, M, N, or R,
n15 represents an integer of from 0 to 2, preferably 1,
R³¹ represents A, C, D, E, F, G, H, I, K, L, M, N, P, Q, R, S, T, V, W, or Y, preferably C, E, F, I, K, L, or W, and
n16 represents an integer of from 0 to 7, preferably from 1 to 6.

More specifically, the three-finger toxin is, for example, a polypeptide including an amino acid sequence set forth in SEQ ID NO: 1 or an amino acid sequence obtained by substituting, deleting, or adding one or a plurality of amino acids in the amino acid sequence set forth in SEQ ID NO: 1:

SEQ ID NO: 1:
MKTLLLILGVVAFVYLEPGYTRKCCIYPKSALFCREVQICPADEGMCFKMWNSTDRLMERTYVRG
CAAACPNPVGDEEVYCCVTDNCNK.

In another preferred embodiment, the three-finger toxin is, for example, a polypeptide including an amino acid sequence set forth in SEQ ID NO: 2 or an amino acid sequence obtained by substituting, deleting, or adding one or a plurality of amino acids in the amino acid sequence set forth in SEQ ID NO: 2:

SEQ ID NO: 2:

MKTLLLILGVVAFVYLDSGFSLICNTCNRTVRIPLIPCDPVTCPEGLDQCYANRTGILRMKVERG

CTTNCTQTLTLMCCKRDMCN.

In another preferred embodiment, the three-finger toxin is, for example, a polypeptide including an amino acid sequence set forth in SEQ ID NO: 3 or an amino acid sequence obtained by substituting, deleting, or adding one or a plurality of amino acids in the amino acid sequence set forth in SEQ ID NO: 3:

SEQ ID NO: 3:
MKTHLLALVMVAFLYLEPGYTLRCHSCVGYVCKEFEFCKPEQKQCIYLINRKKYSGCSRNCPSAG
ANELVKCCATDRCNLEIFCW.

In another preferred embodiment, the three-finger toxin is, for example, a polypeptide including an amino acid sequence set forth in SEQ ID NO: 4 or an amino acid sequence obtained by substituting, deleting, or adding one or a plurality of amino acids in the amino acid sequence set forth in SEQ ID NO: 4:
SEQ ID NO: 4:
MKTLLLILGVVAFVYLDSGFSLKCEQCNLPNCDFLPDVRCPKGLDQCYKKWNETAKVFERGCTAN CNEDEQTRCCKRNGCNF. In such embodiment, it is preferred that the three-finger toxin be, for example: a polypeptide including an amino acid sequence set forth in SEQ ID NO: 1 or an amino acid sequence obtained by substituting, deleting, or adding one or a plurality of amino acids in the amino acid sequence set forth in SEQ ID NO: 1; a polypeptide including an amino acid sequence set forth in SEQ ID NO: 2 or an amino acid sequence obtained by substituting, deleting, or adding one or a plurality of amino acids in the amino acid sequence set forth in SEQ ID NO: 2; or a polypeptide including an amino acid sequence set forth in SEQ ID NO: 4 or an amino acid sequence obtained by substituting, deleting, or adding one or a plurality of amino acids in the amino acid sequence set forth in SEQ ID NO: 4.

In the present invention, the "one or a plurality of" amino acids are, for example, 1 to 20, 1 to 10, 1 to 5, 1 to 3, or 1 or 2 amino acids. Although the position at which the amino acid is substituted, deleted, or added in each of SEQ ID NOS: 1 to 4 is not limited, the position is, for example, a position except those of the amino acids conserved in each of SEQ ID NOS: 1 to 4 such as a position except the following positions in the above-mentioned general formula: "MKT" on an N-terminal side; "LL" between R¹ and R²; "L" between R² and R³; "VAF" between R⁴ and R⁵; "YL" between R⁵ and R⁶; "G" between R⁷ and R⁸; "C" between (R¹¹)ₙ₁ and (R¹²)ₙ₂; "C" between (R¹²)ₙ₂ and (R¹³)ₙ₃; "C" between (R¹³)ₙ₃ and (R¹⁴)ₙ₄; "C" between (R¹⁴)ₙ₄ and (R¹⁵)ₙ₅; "P" between (R¹⁵)ₙ₅ and (R¹⁶)ₙ₆; "C" between (R¹⁸)ₙ₇ and (R¹⁹)ₙ₈; "GC" between (R²²)ₙ₁₀ and (R²³)ₙ₁₁; "C" between R²⁴ and (R²⁵)ₙ₁₂; "CC" between (R²⁷)ₙ₁₃ and (R²⁸)ₙ₁₄; and "CN" between (R³¹)ₙ₁₅ and (R³²)ₙ₁₆. More specifically, when the amino acid is substituted, deleted, or added, the position is preferably any position selected from the following underlined portions in SEQ ID NOS: 1 to 4:
SEQ ID NO: 1: 
MKTLLLILGVVAFVYLEPGYTRKCCIYPKSALFCREVQICPADEGMCFKMWNSTDRLMERTYVRG
CAAACPNPVGDEEVYCCVTDNCNK
SEQ ID NO: 2: 
MKTLLLILGVVAFVYLDSGFSLICNTCNRTVRIPLIPCDPVTCPEGLDQCYANRTGILRMKVERG
CTTNCTQTLTLMCCKRDMCN
SEQ ID NO: 3: 
MKTHLLALVMVAFLYLEPGYTLRCHSCVGYVCKEFEFCKPEQKQCIYLINRKKYSGCSRNCPSAG
ANELVKCCATDRCNLEIFCW
SEQ ID NO: 4:
   MKTLLLILGVVAFVYLDSGFSLKCEQCNLPNCDFLPDVRCPKGLDQCYKKWNETAKVFERGCTAN CNEDEQTRCCKRNGCNF. In addition, in another preferred embodiment, the position at which the amino acid is substituted, deleted, or added in each of SEQ ID NOS: 1 to 4 is, for example, a position represented by (R¹¹)ₙ₁, (R¹²)ₙ₂, (R¹³)ₙ₃, (R¹⁴)ₙ₄, (R¹⁵)ₙ₅, (R¹⁶)ₙ₆, (R¹⁸)ₙ₇, (R¹⁹)ₙ₈, (R²¹)ₙ₉, (R²³)ₙ₁₁, (R²⁵)ₙ₁₂, (R²⁷)ₙ₁₃, (R²⁸)ₙ₁₄, (R³¹)ₙ₁₅, or (R³²)ₙ₁₆ in the above-mentioned general formula. More specifically, when the amino acid is substituted, deleted, or added, the position is preferably any position selected from the following underlined portions in SEQ ID NOS: 1 to 4:
SEQ ID NO: 1: 
MKTLLLILGVVAFVYLEPGYTRKCCIYPKSALFCREVQICPADEGMCFKMWNSTDRLMERTYVRG
CAAACPNPVGDEEVYCCVTDNCNK
SEQ ID NO: 2: 
MKTLLLILGVVAFVYLDSGFSLICNTCNRTVRIPLIPCDPVTCPEGLDQCYANRTGILRMKVERG
CTTNCTQTLTLMCCKRDMCN
SEQ ID NO: 3: 
MKTHLLALVMVAFLYLEPGYTLRCHSCVGYVCKEFEFCKPEQKQCIYLINRKKYSGCSRNCPSAG
ANELVKCCATDRCNLEIFCW
SEQ ID NO: 4:

MKTLLLILGVVAFVYLDSGFSLKCEQCNLPNCDFLPDVRCPKGLDQCYKKWNETAKVFERGCTAN
CNEDEQTRCCKRNGCNF.

As described above, in the present invention, not only a polypeptide including an amino acid sequence set forth in any one of SEQ ID NOS: 1 to 4 but also a polypeptide including an amino acid sequence obtained by substituting, deleting, or adding one or a plurality of amino acids in the amino acid sequence set forth in any one of SEQ ID NOS: 1 to 4 to the extent that the polypeptide has γ-secretase inhibitory activity may be used. The γ-secretase inhibitory activity may be measured by, for example, a method described in Examples to be described later. More specifically, the presence or absence of the γ-secretase inhibitory activity may be evaluated by, for example, a β-galactosidase assay to be described later in accordance with whether or not β-galactosidase activity can be significantly reduced as compared to a WT (without any 3FTX) (more preferably, whether or not the β-galactosidase activity can be reduced to an extent equal to or more than a Pf3FTX-2 does).

The three-finger toxin serving as the active ingredient in the pharmaceutical of the present invention may be produced by a method known per se, such as a genetic engineering-based method or organic synthetic chemical peptide synthesis such as a solid-phase synthesis method.

In the present invention, the three-finger toxin may be used alone as a pharmaceutical, or may be used as a pharmaceutical composition in combination with any of various pharmaceutically acceptable carriers (e.g., a tonicity agent, a chelating agent, a stabilizing agent, a pH regulator, a preservative, an antioxidant, a solubilizing agent, or a thickening agent).

Examples of the tonicity agent include: sugars, such as glucose, trehalose, lactose, fructose, mannitol, xylitol, and sorbitol; polyhydric alcohols, such as glycerin, polyethylene glycol, and propylene glycol; and inorganic salts, such as sodium chloride, potassium chloride, and calcium chloride.

Examples of the chelating agent include: edentates, such as disodium edetate, calcium disodium edetate, trisodium edetate, tetrasodium edetate, and calcium edetate; ethylenediaminetetraacetates; nitrilotriacetic acid or salts thereof; sodium hexametaphosphate; and citric acid.

An example of the stabilizing agent is sodium hydrogen sulfite.

Examples of the pH regulator include acids, such as hydrochloric acid, carbonic acid, acetic acid, and citric acid, and also include: alkali metal hydroxides, such as sodium hydroxide and potassium hydroxide; alkali metal carbonates or hydrogen carbonates such as sodium carbonate; alkali metal acetates such as sodium acetate; alkali metal citrates such as sodium citrate; and bases such as trometamol.

Examples of the preservative include: sorbic acid; potassium sorbate; parahydroxybenzoates, such as methyl parahydroxybenzoate, ethyl parahydroxybenzoate, propyl parahydroxybenzoate, and butyl parahydroxybenzoate; quaternary ammonium salts, such as chlorhexidine gluconate, benzalkonium chloride, benzethonium chloride, and cetylpyridinium chloride; alkylpolyaminoethylglycine; chlorobutanol; polyquad; polyhexamethylene biguanide; and chlorhexidine.

Examples of the antioxidant include sodium hydrogen sulfite, dried sodium sulfite, sodium pyrosulfite, and concentrated mixed tocopherols.

Examples of the solubilizing agent include sodium benzoate, glycerin, D-sorbitol, glucose, propylene glycol, hydroxypropyl methylcellulose, polyvinylpyrrolidone, macrogol, and D-mannitol.

Examples of the thickening agent include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and polyvinyl alcohol.

In addition, the above-mentioned pharmaceutical composition may further include a drug that is known to have a preventive or therapeutic effect on dementia in addition to the three-finger toxin. Examples of the drug that is known to have a preventive or therapeutic effect on dementia include: symptom-alleviating drugs that act on neurotransmission, such as Aricept (generic name: donepezil), Exelon Patch or Rivastach (generic name: rivastigmine), Reminyl (generic name: galantamine), and Memary (generic name: memantine); and antibody pharmaceuticals including Aduhelm (generic name: aducanumab) rapidly approved by the United States FDA as an antibody therapeutic drug that removes amyloid β (in addition thereto, lecanemab and donanemab are designated as FDA breakthrough therapeutic drugs and being subjected to clinical trials).

In the embodiment of the pharmaceutical composition, the content of the three-finger toxin in the composition is not particularly limited, and may be appropriately set within, for example, conditions, such as 90 mass% or more, 70 mass% or more, 50 mass% or more, 30 mass% or more, 10 mass% or more, 5 mass% or more, and 1 mass% or more.

A dosage form is not particularly limited, and examples thereof may include various dosage forms including: orally administered agents, such as a tablet, a pill, a capsule, a powder, a granule, a syrup, and a sublingual tablet; and parenterally administered agents, such as an injection (e.g., intravenous injection, intramuscular injection, or local injection), a gargle, a drop, external preparations (an ointment, a cream, a patch, and an inhalant), and a suppository. Of the dosage forms, for example, an injection is preferred.

The content of the three-finger toxin of the present invention in a dose cannot be uniquely defined because the content varies depending on, for example, an administration route, and the age, body weight, and symptom of a patient. However, the daily dose of the three-finger toxin only needs to be typically from about 10 mg to about 5,000 mg, more preferably from about 100 mg to about 1,000 mg. When administered once daily, the three-finger toxin only needs to be contained in the above-mentioned amount in a single dose. When administered three times daily, the three-finger toxin only needs to be contained in an amount corresponding to one-third of the above-mentioned amount in a single dose.

The pharmaceutical of the present invention is administered to patients such as mammals. Examples of the mammals include humans, monkeys, mice, rats, rabbits, cats, dogs, pigs, cattle, horses, and sheep.

The pharmaceutical of the present invention suppresses the production of an Aβ-peptide through γ-secretase inhibition, to thereby alleviate the symptom of dementia. Accordingly, the present invention also provides a γ-secretase inhibitor including a three-finger toxin and an Aβ-peptide production inhibiitor including a three-finger toxin. The active ingredients, dosage forms, doses, and the like of the γ-secretase inhibitor and the Aβ-peptide production inhibiitor are the same as those of the pharmaceutical of the present invention.

Each of the γ-secretase inhibitor and Aβ-peptide production inhibiitor of the present invention may be applied to a living organism, or may be used in vitro. In such embodiment, for example, when the three-finger toxin is caused to act on a γ-secretase in vitro, the γ-secretase is inhibited, and hence Aβ-peptide production can be suppressed. In a preferred embodiment, for example, a cell that produces the γ-secretase may be used. Examples of the cell include a microbial cell and an animal cell. Examples of the microbial cell include yeast, *Escherichia coli,* and Aspergillus (e.g., koji mold). In a preferred embodiment, the inhibition of the γ-secretase by the three-finger toxin can be caused by culturing such cell as follows: the cell is transformed so as to express the γ-secretase, more preferably transformed so as to express the γ-secretase and the three-finger toxin. In addition, the Aβ-peptide production can be suppressed by adding a precursor peptide of the Aβ-peptide as a substrate to a reaction system. A wide variety of media to be used in the culture of the cell may each be used as a medium to be used in the culture. For example, in the case of the yeast, a YPD medium, a SD medium, or the like may be used, and in the case of the *Escherichia coli,* an LB medium, a 2XYT medium, or the like may be used. In addition, an amino acid, an antibiotic, a sugar, or the like may be appropriately added to the medium. The measurement of the activity of the γ-secretase and the measurement of the amount of the Aβ-peptide may each be performed by using a method known per se. γ-Secretase inhibitory activity and an Aβ-peptide production-suppressing effect exhibited by the three-finger toxin can be evaluated by subjecting a sample after the above-mentioned culture to measurement. In addition, in a preferred embodiment of the present invention, a three-finger toxin peptide is reduced in Notch signaling-suppressing effect. Notch is a receptor that plays an important role in the differentiation of a cell and the determination of the fate thereof. Accordingly, some of the related-art γ-secretase inhibitors cause side effects through the inhibition of a Notch signal, and such point has been a problem. The embodiment of the present invention is preferred because its Notch signaling-suppressing (inhibiting) effect is suppressed as compared to those of the related-art γ-secretase inhibitors (e.g., begacestat). The Notch signaling-suppressing (inhibiting) effect may be evaluated through, for example, the measurement of Notch cleavage activity by a method described in Examples of the present application.

Specific embodiments of the present invention are more specifically described below by way of Examples, but the present invention is not limited to Examples below.

### Examples

In this Example, a three-finger toxin peptide may be referred to as "disulfide-rich peptide (DRP peptide)."

### Example 1

The inventors of the present invention have introduced the genes of the four subunits (PS1, NCT, AphlaL, and Pen2) of a human γ-secretase and an amyloid precursor protein (APP) into yeast *(Saccharomyces cerevisiae)* to construct a γ-secretase-expressing system that measures the APP decomposition activity of the γ-secretase (Non-patent Literatures 1 to 3). In Example 1 below, an inhibition evaluation was performed by the following method on the basis of such method.

### [Experiment Operation]

### Inhibition Evaluation of Protobothrops Flavoviridis 3FTX by γ-secretase-expressing System using Human Amyloid Precursor Protein (APP) as Substrate

### <Construction of Expression Vector>

An expression vector: A pBEVY plasmid has bidirectional promoters (an ADH1 promoter and a GPD promoter). Three kinds of plasmids pBEVY-T, pBEVY-L, and pBEVY-U (Trp, Leu, and Uracil, respectively) different from each other in gene to be used in the auxotrophic marker of yeast were used.

Those produced on the basis of a previous report (Non-patent Literature 1) were used as plasmids expressing four subunits (PS1, NCT, Pen2, and Aph1) for forming a γ-secretase and a human amyloid precursor protein (APP) in yeast. Specifically, the PS1 and the NCT were inserted into the Kpn I and Xba I sites of the pBEVY-T vector, respectively (PS1/NCT pBEVY-T), and the Flag-Pen2 and the Aph1-HA were inserted into the Kpn I and Xba I sites of the pBEVY-L vector, respectively (Flag-Pen2/Aph1-HA pBEVY-L). Genes encoding an artificial substrate (hereinafter referred to as "APP_{C55}"), which was obtained by linking regions corresponding to the amino acid residues 672 to 726 of the APP to the endoplasmic reticulum signal sequence (corresponding to amino acid residues 1 to 19) of a yeast invertase Suc2, and an artificial substrate (hereinafter referred to as "APP_{C55}-Gal"), which was obtained by further linking a transcription factor Gal4p to the APP_{C55}, were inserted into the BamHI and EcoRI sites of a p426ADH vector, respectively (APP_{C55} p426ADH and APP_{C55}-Gal p426ADH). In addition, the APP_{C55}-Gal p426ADH was treated with BamHI and EcoRV, and was then treated with Klenow (Takara Bio Inc.) to be blunted, followed by its insertion into the SmaI site of the pBEVY-U (APP_{C55}-Gal pBEVY-U).

Plasmids expressing the genes of *Protobothrops flavoviridis* three-finger toxins (Pf3FTX-1, Pf3FTX-2, Pf3FTX-3, and Pf3FTX-4) in yeast were prepared as described below. The genes of three-finger toxins CFP-Pf3FTX-1, CFP-Pf3FTX-2, CFP-Pf3FTX-3, and CFP-Pf3FTX-4 each having CFP added to an amino terminal, and the APP_{C55}-Gal pBEVY-U plasmid were amplified by PCRs using the following primers, and were purified with MinElute Gel Extraction kit (QIAGEN), followed by their cloning by a recombination reaction using In-Fusion kit (Takara Bio Inc.) (CFP-Pf3FTX-1/APP_{C55}-Gal pBEVY-U, CFP-Pf3FTX-2/APP_{C55}-Gal pBEVY-U, CFP-Pf3FTX-3/APP_{C55}-Gal pBEVY-U, and CFP-Pf3FTX-4/APP_{C55}-Gal pBEVY-U).

**Table 1**

| | |
|---|---|
| Inf-Wbp1_F | 5'-CTTGTTCTTTACGACCATCATCATCATCATCATATG-3' |
| Inf-Wbp1_R | 5'-GACTCTAGAGGATCCGAATTCGGATCCCTCGAG-3' |
| Inv-pBEVY_F | 5'-GTCGTAAAGAACAAGCGTTCTTG-3' |
| Inv-pBEVY_R | 5'-GGATCCTCTAGAGTCGACCTGC-3' |

### <γ-Secretase Activity Evaluation using Establishment and Growth of Yeast Strain as Indicators>

A yeast PJ69-4A (MATa trp1-901 leu2-3, 112 ura3-52 his3-200 gal4Δ gal80Δ LYS::GAL1-HIS3 GAL2-ADE2 met::GAL7-lacZ) strain was selected as a host cell, and DRP peptide-expressing cells were established by transforming the three kinds of expression vectors produced in the foregoing. A detailed method is as described below.

The plasmids produced in the foregoing section were introduced into the PJ69-4A strain in accordance with the following combination: PS1/NCT pBEVY-T, Flag-Pen2/Aph1-HA pBEVY-L, APP-Gal4 pBEVY-U, or APP-Gal4/3FTX pBEVY-U. A lithium acetate method was used in transformation into the yeast. Specifically, the yeast grown in a YPAD up to an O.D.₆₀₀ of from 0.8 to 1.0 was centrifuged (3,000 rpm, 5 minutes, room temperature) and recovered, followed by its suspension in 0.1 M LiOAc. The suspended yeast was centrifuged and recovered again, and 240 µl of 50% PEG, 36 µL of 1 M LiOAc, denatured ssDNA (2 mg/ml), and the plasmid diluted with 50 µL of sterile water, the plasmid being used in an amount corresponding to from 0.1 µg to 10 µg, were superposed on the precipitate. The mixture was stirred with a vortex mixer for 1 minute, and was then incubated at 30°C for 30 minutes and at 42°C for 25 minutes. After that, the incubated product was centrifuged (7,000 rpm, 1 minute, room temperature), and cells were recovered and then suspended in sterile water. An appropriate amount of the suspension was seeded in a SD selective medium (SD-LWU) devoid of Trp, Leu, and Uracil, and was cultured at 30°C for 3 days to provide a transformant.

In the yeast having introduced thereinto such vector, the γ-secretase is formed, and the Gal4 is liberated by the cleavage of the APP_{C55}-Gal. Thus, a reporter gene (HIS3, ADE2, or lacZ) is expressed. The expression of the reporter gene was evaluated by the growth of the yeast on a SD medium (SD-LWHUAde) plate devoid of Trp, Leu, Uracil, His, and Adenine.

### <γ-Secretase Activity Evaluation by β-Galactosidase Assay>

The yeast expressing the γ-secretase complex, the substrate APP_{C55}-Gal4, and the three-finger toxin produced in the foregoing was inoculated in 1.5 mL of a SD-LWU medium, and the resultant was shake-cultured (180 rpm) at 30°C until its turbidity became from 0.8 to 1.0. After that, the cultured product was centrifuged (15,000 rpm, 3 minutes, room temperature), and cells were recovered. The cells were washed with 1 mL of cooling water, and were then suspended in 30 µL of a Lysis buffer (20 mM Tris-HCl, pH: 8.0, 10 mM MgCl₂, 50 mM KCl, 1 mM EDTA, 5% glycerol, and 1 mM DTT), followed by the addition of an appropriate amount of acid-treated glass beads (425 µm to 600 µm, Sigma-Aldrich Co. LLC) thereto. After that, the mixture was stirred with a vortex mixer so that the cells were crushed. The crushed liquid was centrifuged (15,000 rpm, 10 minutes, 4°C) to provide a lysate. 80 Microliters of 4 mg/ml ortho-nitrophenyl-β-galactoside (ONPG) was added to the mixed liquid of 10 µL of the lysate and 400 µL of a Z buffer (60 mM Na₂HPO₄, 40 mM NaH₂PO₄-2H₂O, 10 mM KCl, 1 mM MgSO₄-7H₂O, and 0.27% 2-mercaptoethanol) to start a reaction. Incubation was performed until the reaction liquid showed a yellow color, and 200 µL of 1 M Na₂CO₃ was added to stop the reaction. After that, the absorbance (A₄₂₀) of nitrophenol produced in the reaction liquid at 420 nm was measured. Further, the protein concentration of the lysate was determined by a Bradford method (Bio-Rad Laboratories, Inc.), and β-galactosidase activity was calculated [β-galactosidase unit/mg protein=1,000×A₄₂₀{time (min)×protein concentration (mg/µL)×liquid amount (µL)}]. In addition, a SDS-sample buffer was added to the lysate to turn the lysate into SDS. The resultant sample was electrophoresed with a 12.5% acrylamide gel, and was analyzed by a western blotting method.

### Inhibition Evaluation by In Vitro Aβ Production Assay

### <Preparation of Membrane Fraction from Yeast>

The yeast expressing the γ-secretase complex and the substrate C55 thereof produced in the foregoing was inoculated in 2 L of a SD-LWU medium, and the resultant was shake-cultured (180 rpm) at 30°C until its turbidity became 1. After that, the cultured product was centrifuged (5,000 rpm, 5 minutes, room temperature), and cells were recovered. The cells were suspended in 50 mL of a Tris/DTT buffer (100 mM Tris-HCl, pH: 9.4, and 10 mM dithiothreitol [DTT]), and were centrifuged again, followed by the recovery of the cells. After that, the cells were suspended in 50 mL of a lyticase buffer (0.75% yeast extract, 1.5% peptone, 0.7 M sorbitol, 0.5% glucose, 10 mM Tris-HCl, pH: 7.6, and 1 mM DTT), and Zymolyase-100T (Nacalai Tesque, Inc.) was added thereto, followed by incubation at 30°C for 1 hour. Yeast (spheroplast) whose cell wall had been broken was centrifuged (6,200 rpm, 10 minutes, 4°C) and recovered, and the precipitate was washed with 20 mL of a 2xJR buffer. The washed product was centrifuged again, and the recovered spheroplast was suspended in 5 mL of a 2xJR buffer (40 mM HEPES, pH: 7.4, 0.4 M sorbitol, 100 mM KOAc, and 4 mM EDTA). The suspension was frozen at -80°C, and was then dissolved on ice. 10 Milliliters of cooled water, 20 µL of 1 M DTT, 20 µL of a protease inhibitor cocktail-I (aqueous solution of 1 M p-aminobenzamidine dihydrochloride, 0.1% antipain dihydrochloride, 0.1% aprotinin, and 0.1% leupeptin), and 20 µL of a protease inhibitor cocktail-II (DMSO solution of 0.1% chymostatin, 1 M PMSF, 1 M TPCK, and 0.1% leupeptin) were added to the dissolved product, and the materials were mixed. The mixture was crushed with a 30 mL Potter Elvehjem homogenizer (Wheaton Industries) (6,500 rpm, 20 strokes), and the resultant crushed liquid was centrifuged (3,000 rpm, 5 minutes, 4°C), followed by the removal of uncrushed cells and nuclei. The supernatant was centrifuged again (15,000 rpm, 10 minutes, 4°C) to provide a precipitate containing a microsome. The precipitate was suspended in 3 mL of a B88 buffer (20 mM HEPES, pH: 6.8, 250 mM sorbitol, 150 mM KOAc, and 5 mM Mg(OAC)₂), and the suspension was superposed on a concentration gradient produced by using 2.4 mL of a 1.5 M sucrose solution and 2.4 mL of a 1.2 M sucrose solution. The resultant was centrifuged (45,000 rpm, 100 minutes, 4°C: CX70MX Ultracentrifuge manufactured by Hitachi, Ltd., rotor: P40ST) to be fractionated into the following three fractions: a lipid (0 M sucrose), a vacuole (0 M to 1.2 M sucrose), and a microsome (1.2 M to 1.5 M sucrose). The lipid and the vacuole were removed, and the microsome fractionated into the 1.2 M to 1.5 M sucrose was recovered, and was diluted with a gamma buffer (50 mM PIPES, pH: 7.0, 250 mM sucrose, and 1 mM EGTA) whose amount was three times as large as that of the microsome, followed by its centrifugation (15,000 rpm, 10 minutes, 4°C). The precipitate was suspended in the gamma buffer again, and was stored at -80°C until its use. Part of the suspension was subjected to protein concentration measurement with a DC protein assay kit (Bio-Rad Laboratories, Inc.).

### <Cell-free Assay System using Membrane Fraction of Yeast>

As in previous research (Non-patent Literature 3) using the membrane fraction of a cell, a cell-free assay for observing γ-secretase activity was performed with the membrane fraction of yeast. The membrane fraction collected in the foregoing was incubated on ice in a gamma buffer containing 1% CHAPSO for 1 hour to be solubilized, and was then diluted with a gamma buffer so that the concentration of CHAPSO became 0.25% and its protein concentration became 400 µg/mL. At this time, various protease inhibitors were added to the fraction so that their final concentrations were as follows: 50 µM diisopropyl fluorophosphate, 50 µM phenylmethylsulfonyl fluoride, 0.1 µg/mL N^{α}-p-tosyl-L-lysine chloromethyl ketone, 0.1 µg/mLantipain, 0.1 µg/mL leupeptin, 100 µM EGTA, 1 mM thiorphan, and 5 mM phenanthroline. Further, three-finger toxins (3FTX-1, 3FTX-2, 3FTX-3, and 3FTX-4) purified with *Escherichia coli* by the above-mentioned method were each added to the mixture so as to have a final concentration of from 1 µM to 10 µM. As required, DAPT (Peptide Institute, Inc.) widely used as a γ-secretase inhibitor was added to the mixture so as to have a final concentration of 1 mM, and phosphatidyl choline (PC) (Sigma-Aldrich Co. LLC) was added thereto so as to have a final concentration of 0.1%, followed by the setting of the amount of the mixed reaction liquid to 200 µL.

The temperature of the reaction liquid was held at 37°C for 24 hours to cause an Aβ production reaction by the γ-secretase. 500 Microliters of a liquid obtained by mixing chloroform and methanol at 2:1 was added to the reaction liquid to stop the reaction. After the liquids had been mingled with each other, the mixture was left at room temperature for 1 hour, and 500 µL of methanol was further added thereto. After the liquids had been mingled with each other by being inverted, the mixture was centrifuged (13,000 rpm, 20 minutes, 4°C), and 300 µL of a liquid obtained by mixing chloroform, methanol, and water at 1:2:0.8 was added to the precipitate, followed by mixing. After that, the mixture was similarly centrifuged again, and the precipitate was recovered. The precipitate was air-dried at room temperature for 1 day. Thus, delipidation was performed, and hence a protein was able to be extracted. 40 Microliters of a 1xSDS sample buffer was added to the air-dried precipitate, and the mixture was incubated at 100°C for 5 minutes to denature the protein. Thus, a sample for western blotting was obtained.

### <Western Blotting for detecting Aβ>

An Aβ was detected with the above-mentioned sample by the following method. Gel plates each measuring 10 cm long by 10 cm wide, and having a 1-millimeter spacer were used, and a 16.5% acrylamide gel (6 mL) was solidified as a separation gel. After that, an appropriate amount of a 4.7% acrylamide gel serving as a stacking gel was superposed thereon, and a comb was inserted thereinto, followed by the solidification of the gel. 1 M Tris-HCl [pH: 8.45] and 0.03% SDS were added to each of the gels. Electrophoresis was performed in a Tris-Tricine system using a cathode buffer (0.1 M Tris-HCl [pH: 8.25], 0.1 M Tricine, and 0.1% SDS) on a cathode side and an anode buffer (0.2 M Tris-HCl [pH: 8.9]) on an anode side, and the protein was separated as follows: while a phoresis tip was present in the stacking gel, a constant voltage of 40 V was applied; and after the tip had entered the separation gel, a constant current of 35 mA was applied per gel plate. The unfolded protein was transferred onto a nitrocellulose membrane (at 150 mA for 2 hours and 30 minutes) with a TE22 Mighty Small Transphor tank-type transfer system (Cytiva). The nitrocellulose membrane after the transfer was boiled in boiling PBS for 5 minutes, and was then immersed in TBS-T containing 5% skim milk for 1 hour, followed by its washing in TBS-T. The nitrocellulose membrane was immersed in an antibody diluted liquid, which was obtained by diluting an antibody 82E1 (Immuno-Biological Laboratories Co., Ltd.) specific to the N-terminal of the Aβ with TBS-T so that its concentration became 1/1,000, and the membrane was incubated at 4°C overnight. After having been washed in TBS-T, the nitrocellulose membrane was immersed in a secondary antibody diluted liquid, which was obtained by diluting an anti-mouse IgG, HRP-linked antibody (CST) with TBS-T so that its concentration became 1/5,000, and the membrane was incubated at room temperature for 1 hour. After having been washed in TBS-T, the membrane was caused to develop a color with an enhanced chemiluminescence (ECL) system, and then the Aβ was detected with LAS-3000 (FUJIFILM Corporation). The molecular weight of the detected protein was identified with an Aβ40 peptide (Peptide Institute, Inc.) and Precision Plus Protein Dual Extra Standard (Bio-Rad Laboratories, Inc.).

### Expression and Purification of Three-finger Toxin with Escherichia Coli

### <Construction of Expression Vector>

Synthetic genes that had already been optimized for the codon of *Escherichia coli* were used as the respective genes of the Pf3FTX-1 to the Pf3FTX-4. A Nde I cleavage site and an Xho I cleavage site were introduced into the 5'-terminal and 3'-terminal of each of the genes, respectively, and the resultant was incorporated into a pCold I vector or a pCold II vector by restriction enzyme treatment (Pf3FTX/pCold I or Pf3FTX/pCold II: the products were each produced for the four kinds, that is, the Pf3FTX-1 to the Pf3FTX-4). Further, a CFP gene and the 3FTX/pCold I vectors were amplified by PCRs using the following primers, and were purified with MinElute Gel Extraction kit (QIAGEN), followed by their cloning by a recombination reaction using In-Fusion kit (Takara Bio Inc.) (CFP-Pf3FTX-1/pCold I, CFP-Pf3FTX-2/pCold I, CFP-Pf3FTX-3/pCold I, and CFP-Pf3FTX-4/pCold I).

**Table 2**

| | |
|---|---|
| insert-CFP-pCold-F | 5'-CACAAAGTGCATCATCATCATCATCATATGGTGAGCAAGGGCG-3' |
| insert-CFP-pCold-R | 5'-ATGCCTACCTTCGATGTACAGCTCGTCCATG-3' |
| GST-pCold vector F | 5'-ATCGAAGGTAGGCATATG-3' |
| vector-CFP-pCold-R | 5'-ATGATGATGATGATGATGCACTTTGTGATTC-3' |

### <Expression and Purification in Escherichia Coli>

The produced plasmids for expression were each transformed into an E. *coli* BL21 strain, and the resultant was used as a target protein-expressing strain. The expression of a protein was performed as described below. Each of the expressing strains was grown in 2 L of a 2×YT ampicillin medium (2.5% tryptone, 1.5% yeast extract, 0.5% NaCl, and 100 µg/mL ampicillin) up to an O.D.₆₀₀ of 0.6, and then isopropyl-β-thiogalactopyranoside (IPTG) was added thereto so as to have a final concentration of 0.1 mM, followed by the induction of the expression at 15°C for 24 hours. Cells were recovered by centrifugation (8,000 rpm, 5 minutes, 4°C), and were then suspended in 25 mL of a sonication buffer (50 mM Tris-HCl (pH: 9.0) and 300 mM NaCl) with respect to 500 mL of a culture solution. The suspension was subjected to ultrasonic crushing treatment by performing the following cycle for 15 minutes: the suspension was crushed with an ultrasonic generator UD-200 (Tomy Seiko Co., Ltd.) for 30 seconds while being cooled with ice; and the crushing was halted for 30 seconds. The crushed solution was centrifuged (15,000 rpm, 30 minutes, 4°C), and the supernatant was recovered as a soluble fraction. The soluble fraction after the extraction of the protein was passed through a 0.45-micrometer filter Millex-HP (Merck Millipore) so that its fine particles were removed. The residue was used as a crude extract. The purification of the protein was performed with a HisTrap HP 5 mL affinity column (Cytiva) by an operation using AKTA Start (GE HealthCare). A specific operation procedure of the AKTA Start was as follows: (1) sample loading: the affinity column was replaced with the sonication buffer whose amount was 10 times as large as that of the column, and then the crude protein liquid was flowed at a flow rate of 2 mL/min to be caused to adsorb to a carrier; (2) the removal of a nonspecifically binding protein: until a UV absorption value returned to a baseline, a protein nonspecifically binding to the carrier was removed by flowing a washing buffer (50 mM Tris-HCl (pH: 9.0), 300 mM NaCl, and 10 mM imidazole) at a flow rate of 3 mL/min; and (3) the elution of a target protein: an elution buffer (50 mM Tris-HCl (pH: 9.0), 300 mM NaCl, and 50 mM to 500 mM imidazole) whose imidazole concentration was increased in a stepwise manner to 50 mM, 100 mM, 200 mM, 300 mM, and 500 mM was flowed at a flow rate of 3 mL/min. A fraction whose peak was able to be observed from the UV absorption value was recovered, and the fraction was used as a purified protein liquid. The target protein was identified by subjecting the purified fraction to electrophoresis by SDS-PAGE in amounts of 5 µL each and subjecting the fraction to CBB staining.

The fraction in which the presence of the target protein was able to be recognized was subjected to desalting and concentration by an ultrafiltration method. The protein solution was added to Microsep Advance Centrifugal Devices 1K Omega (Pall Corporation) or Amicon Ultra-4 (Merck Millipore, MWCO=10,000), and was centrifuged (2,750 rpm, 30 minutes, 4°C) a plurality of times. At this time, the removal of imidazole and the concentration of the protein were performed by adding the sonication buffer.

### [Result]

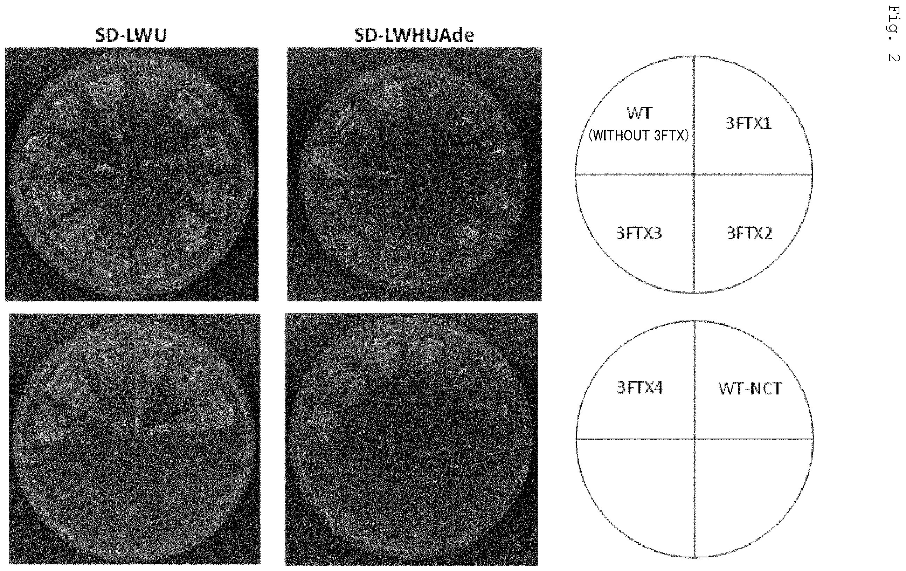

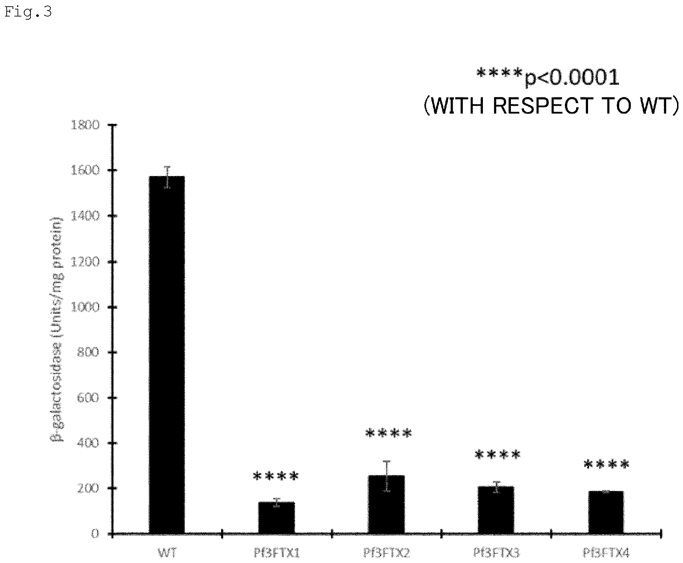

In the yeast expressing the human γ-secretase complex formed of the four subunits (PS1, NCT, Pen2, and Aph1) and the human amyloid precursor protein (APP), the APP is recognized, and hence the Gal4 transcription factor artificially added as a fusion protein to the C-terminal side of the APP is cleaved and liberated. Thus, the reporter gene (HIS3, ADE2, or lacZ) is expressed. As a result, the growth of the yeast was observed even in the SD-LWHUAde plate serving as a nutrient medium free of histidine and adenine (the right of Fig. 2, WT (WITHOUT 3FTX)). However, when each of the *Protobothrops flavoviridis* three-finger toxins (Pf3FTX-1, Pf3FTX-2, Pf3FTX-3, and Pf3FTX-4) was co-expressed, the growth was inhibited to the same level as that of a plate (WT-NCT) devoid of the NCT (Fig. 2). As a result of an evaluation by the enzyme activity of β-galactosidase encoded by a reporter gene, the WT showed a value as high as 1,571 units/mg protein, but in each of the plates in which the *Protobothrops flavoviridis* three-finger toxins (Pf3FTX-1, Pf3FTX-2, Pf3FTX-3, and Pf3FTX-4) were co-expressed, β-galactosidase activity was strongly inhibited as follows (Fig. 3): 137 units/mg in the Pf3FTX-1, 254 units/mg in the Pf3FTX-2, 204 units/mg in the Pf3FTX-3, and 185 units/mg in the Pf3FTX-4.

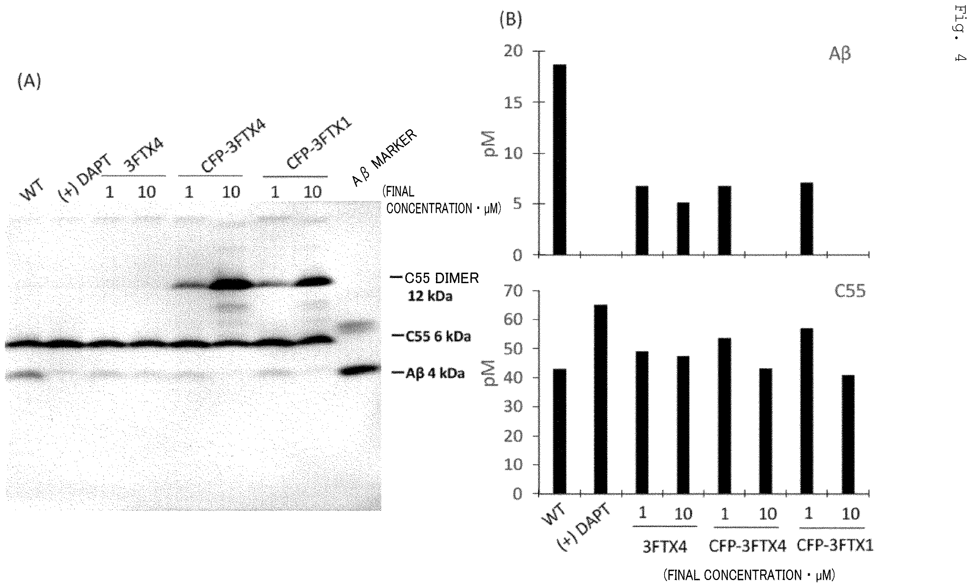

Subsequently, to directly verify the inhibition of a decomposition reaction by a γ-secretase, inhibitory activity was evaluated by an in vitro Aβ production assay. A microsome (40 µg) containing the γ-secretase complex and a microsome (40 µg) containing the APP_{C55} were each solubilized with 1% CHAPSO. After that, phosphatidyl choline (PC) (Sigma-Aldrich Co. LLC) was added to the resultant so as to have a final concentration of 0.1%, and a three-finger toxin (CFP-Pf3FTX-1, CFP-3FTX-4, or 3FTX-4) was added to the mixture, followed by incubation at 37°C. A produced Aβ was analyzed by western blotting. As a result, the addition of the three-finger toxin reduced the production amount of the Aβ in a concentration-dependent manner. The degree of the inhibition was significant in each of the CFP-Pf3FTX-1 and the CFP-3FTX-4, and the inhibition at a concentration of 10 µM was comparable to inhibition by DAPT (5 µM) that was a γ-secretase-specific inhibitor. In addition, the production of a C55 dimer by the addition of the CFP-Pf3FTX-1 or the CFP-3FTX-4 was observed (Fig. 4). As described above, the four kinds of *Protobothrops flavoviridis* three-finger toxin peptides (Pf3FTX-1, Pf3FTX-2, Pf3FTX-3, and Pf3FTX-4) each inhibited the activity by which the APP of the human γ-secretase was decomposed to produce the Aβ.

### Example 2

The inventors of the present invention have introduced the genes of the four subunits (PS1, NCT, AphlaL, and Pen2) of a human γ-secretase and Notch into yeast *(Saccharomyces cerevisiae)* to construct a γ-secretase-expressing system that measures the decomposition activity of the γ-secretase in the case of using the Notch as a substrate (Non-patent Literatures 1 to 3). In Example 2 below, an inhibition evaluation was performed by the following method on the basis of such method.

### [Experiment Operation]

### Inhibition Evaluation of Protobothrops Flavoviridis 3FTX by γ-secretase-expressing System using Notch as Substrate

### <Construction of Expression Vector>

An expression vector: A pBEVY plasmid has bidirectional promoters (an ADH1 promoter and a GPD promoter). Three kinds of plasmids pBEVY-T, pBEVY-L, and pBEVY-U (Trp, Leu, and Uracil, respectively) different from each other in gene to be used in the auxotrophic marker of yeast were used.

Those produced on the basis of a previous report (Non-patent Literature 1) were used as plasmids expressing four subunits (PS1, NCT, Pen2, and Aph1) for forming a γ-secretase and Notch in yeast. Specifically, the PS1 and the NCT were inserted into the Kpn I and Xba I sites of the pBEVY-T vector, respectively (PS1/NCT pBEVY-T), and the Flag-Pen2 and the Aph1-HA were inserted into the Kpn I and Xba I sites of the pBEVY-L vector, respectively (Flag-Pen2/Aph1-HA pBEVY-L). A gene encoding an artificial substrate (hereinafter referred to as "Notch-Gal4"), which was obtained by linking the endoplasmic reticulum signal sequence (corresponding to amino acid residues 1 to 19) of a yeast invertase Suc2, regions corresponding to the amino acid residues 1704 to 1755 of mouse Notch1, and a transcription factor Gal4p to each other, was inserted into the BamHI and EcoRI sites of a p426ADH vector (Notch1-Gal p426ADH).

Plasmids expressing the genes of *Protobothrops flavoviridis* three-finger toxins (Pf3FTX-1, Pf3FTX-2, Pf3FTX-3, and Pf3FTX-4) in yeast were prepared as described below. The genes of three-finger toxins CFP-Pf3FTX-1, CFP-Pf3FTX-2, CFP-Pf3FTX-3, and CFP-Pf3FTX-4 each having CFP added to an amino terminal, and the Notch1-Gal p426ADH plasmid were amplified by PCRs using the following primers, and were purified with MinElute Gel Extraction kit (QIAGEN), followed by their cloning by a recombination reaction using In-Fusion kit (Takara Bio Inc.) (CFP-Pf3FTX-1/Notch-Gal pBEVY-U, CFP-Pf3FTX-2/Notch-Gal pBEVY-U, CFP-Pf3FTX-3/Notch-Gal pBEVY-U, and CFP-Pf3FTX-4/Notch-Gal pBEVY-U) .

**Table 3**

| | |
|---|---|
| Inf-Notch_F | 5'-CCCCGATCCTATATGATGCTTTTGCAAGC-3' |
| Inf-Notch_R | 5'-TAGCTTCATACTAGTGCCATGC-3' |
| Inv-C55Gal_F | 5'-ACTAGTATGAAGCTACTGTCTTC-3' |
| Inv-C55Gal_R | 5'-CATATAGGATCGGGGAGTTGATTG-3' |

### <γ-Secretase Activity Evaluation using Establishment and Growth of Yeast Strain as Indicators>

A yeast PJ69-4A (MATa trp1-901 leu2-3, 112 ura3-52 his3-200 gal4Δ gal80Δ LYS::GAL1-HIS3 GAL2-ADE2 met::GAL7-lacZ) strain was selected as a host cell, and DRP peptide-expressing cells were established by transforming the three kinds of expression vectors produced in the foregoing. A detailed method is as described below.

The plasmids produced in the foregoing section were introduced into the PJ69-4A strain in accordance with the following combination: PS1/NCT pBEVY-T, Flag-Pen2/Aph1-HA pBEVY-L, Notch-Gal4 pBEVY-U, or 3FTX/Notch-Gal4 pBEVY-U. A lithium acetate method was used in transformation into the yeast. Specifically, the yeast grown in a YPAD up to an O.D.₆₀₀ of from 0.8 to 1.0 was centrifuged (3,000 rpm, 5 minutes, room temperature) and recovered, followed by its suspension in 0.1 M LiOAc. The suspended yeast was centrifuged and recovered again, and 240 µL of 50% PEG, 36 µL of 1 M LiOAc, 50 µL of denatured ssDNA (2 mg/ml), and the plasmid diluted with 25 µL of sterile water, the plasmid being used in an amount corresponding to from 0.1 µg to 10 µg, were superposed on the precipitate. The mixture was stirred with a vortex mixer for 1 minute, and was then incubated at 30°C for 30 minutes and at 42°C for 30 minutes. After that, the incubated product was centrifuged (7,000 rpm, 1 minute, room temperature), and cells were recovered and then suspended in sterile water. An appropriate amount of the suspension was seeded in a SD selective medium (SD-LWU) devoid of Trp, Leu, and Uracil, and was cultured at 30°C for 3 days to provide a transformant.

In the yeast having introduced thereinto such vector, the γ-secretase is formed, and the Gal4 is liberated by the cleavage of the Notch-Gal. Thus, a reporter gene (HIS3, ADE2, or lacZ) is expressed. The expression of the reporter gene was evaluated by the growth of the yeast on a SD medium (SD-LWHUAde) plate devoid of Trp, Leu, Uracil, His, and Adenine.

### <γ-Secretase Activity Evaluation by β-Galactosidase Assay>

The yeast expressing the γ-secretase complex, the substrate Notch-Gal4, and the three-finger toxin produced in the foregoing was inoculated in 15 mL of a SD-LWU medium, and the resultant was shake-cultured (180 rpm) at 30°C until its turbidity became from 0.8 to 1.0. After that, a 1.5 mL aliquot of the cultured product was dispensed and centrifuged (15,000 rpm, 3 minutes, room temperature), and cells were recovered. The cells were washed with 1 mL of cooling water, and were then suspended in 30 µL of a Lysis buffer (20 mM Tris-HCl, pH: 8.0, 10 mM MgCl₂, 50 mM KCl, 1 mM EDTA, 5% glycerol, and 1 mM DTT), followed by the addition of an appropriate amount of acid-treated glass beads (425 µm to 600 µm, Sigma-Aldrich Co. LLC) thereto. After that, the mixture was stirred with a vortex mixer so that the cells were crushed. The crushed liquid was centrifuged (15,000 rpm, 10 minutes, 4°C) to provide a lysate. 80 Microliters of 4 mg/ml ortho-nitrophenyl-β-galactoside (ONPG) was added to the mixed liquid of 5 µL of the lysate and 400 µL of a Z buffer (60 mM Na₂HPO₄, 40 mM NaH₂PO₄-2H₂O, 10 mM KCl, 1 mM MgSO₄-7H₂O, and 0.27% 2-mercaptoethanol) to start a reaction. Incubation was performed until the reaction liquid showed a yellow color, and 200 µL of 1 M Na₂CO₃ was added to stop the reaction. After that, the absorbance (A₄₂₀) of nitrophenol produced in the reaction liquid at 420 nm was measured. Further, the protein concentration of the lysate was determined by a Bradford method (Bio-Rad Laboratories, Inc.), and β-galactosidase activity was calculated: β-galactosidase unit/mg protein=1,000 × A420/{time (min) × protein concentration (mg/µL) × liquid amount (µL)}.

In addition, a SDS-sample buffer was added to the lysate to turn the lysate into SDS. The resultant sample was electrophoresed with a 12.5% acrylamide gel, and was analyzed by a western blotting method.

### [Result]

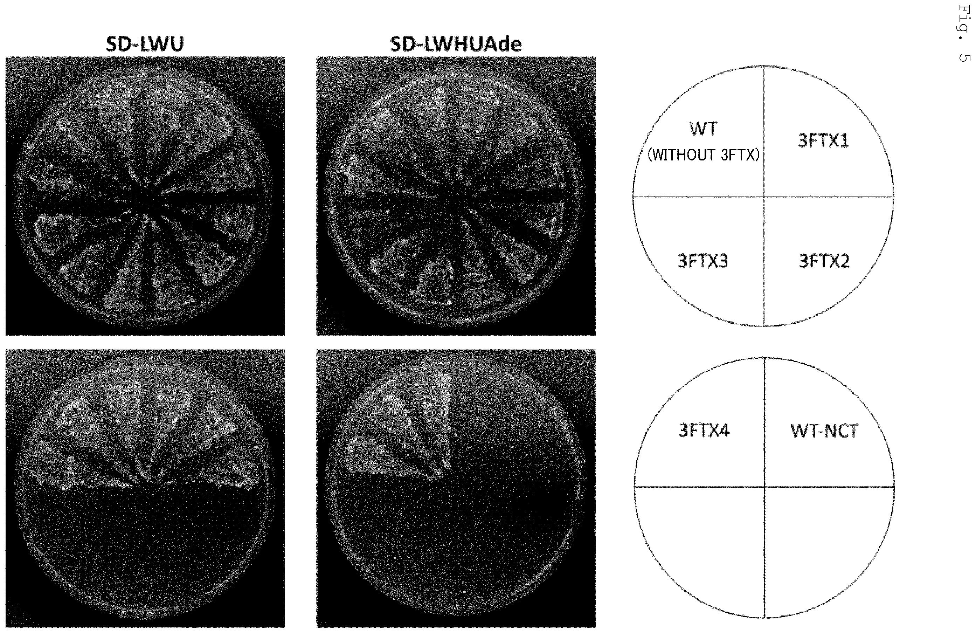

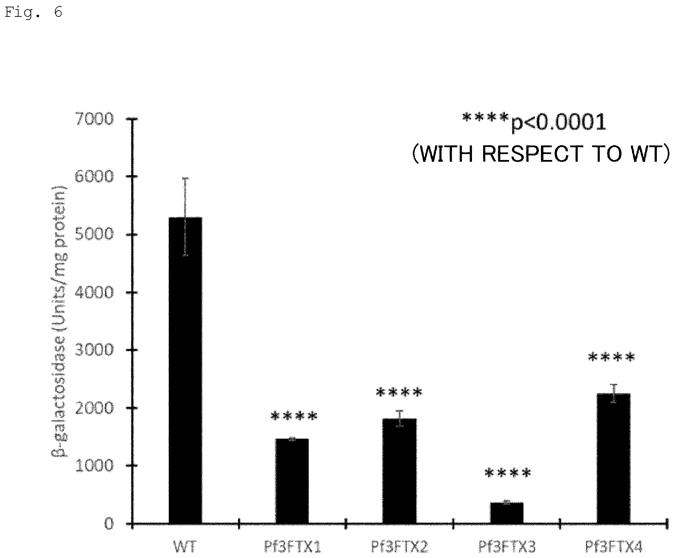

Next, the inhibition of Notch cleavage responsible for the side effect of a γ-secretase inhibitor was analyzed. In yeast expressing the human γ-secretase complex formed of the four subunits (PS1, NCT, Pen2, and Aph1) and the mouse Notch1, the Notch is recognized, and hence the Gal4 transcription factor artificially added as a fusion protein to the C-terminal side of the Notch is cleaved and liberated. Thus, the reporter gene (HIS3, ADE2, or lacZ) is expressed. As a result, in a WT that did not express any *Protobothrops flavoviridis* three-finger toxin (Pf3FTX), the growth of the yeast was observed even in the SD-LWHUAde plate serving as a nutrient medium free of histidine and adenine (the right of Fig. 5, WT (WITHOUT 3FTX)), but the growth was completely inhibited in a plate (WT-NCT) devoid of the NCT (the right of Fig. 5, WT-NCT). Meanwhile, when each of the Pf3FTX-1, the Pf3FTX-2, the Pf3FTX-3, and the Pf3FTX-4 was co-expressed, the growth was inhibited as compared to the WT, but was not completely inhibited unlike the WT-NCT (Fig. 5). As a result of an evaluation by the enzyme activity of β-galactosidase encoded by a reporter gene, the WT showed a value as high as 5,301 units/mg protein, but in each of the plates in which the *Protobothrops flavoviridis* three-finger toxins (Pf3FTX-1, Pf3FTX-2, Pf3FTX-3, and Pf3FTX-4) were co-expressed, β-galactosidase activity was inhibited as follows (Fig. 6): 1,465 units/mg in the Pf3FTX-1, 1,816 units/mg in the Pf3FTX-2, 367 units/mg in the Pf3FTX-3, and 2,250 units/mg in the Pf3FTX-4. It can be said that each of the Pf3FTX-1, the Pf3FTX-2, and the Pf3FTX-4 suppresses the Notch cleavage more weakly than the Pf3FTX-3 does, and is hence excellent as a γ-secretase inhibitor targeting AD.

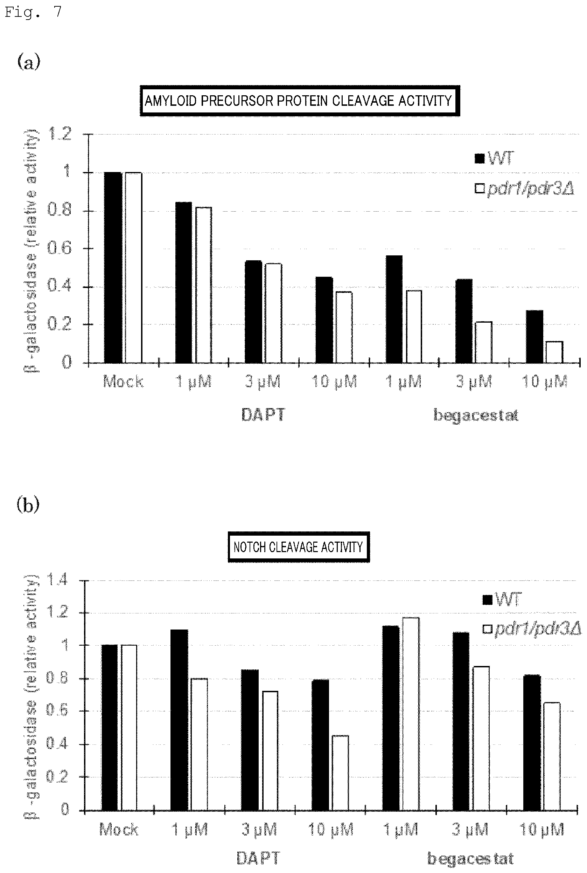

The results were compared to a known APP-specific γ-secretase inhibitor. A known APP-specific γ-secretase inhibitor begacestat was added (at 1 µM, 3 µM, and 10 µM) to a culture solution of yeast expressing the human γ-secretase complex and a gene obtained by fusing the Gal4 to the APP or the Notch1, and its cleavage activity was evaluated by the enzyme activity of β-galactosidase encoded by a reporter gene. As a result of comparison using wild-type yeast and a pdr1Δpdr3Δ gene-knockout strain in which drug efflux was suppressed by blocking the transcription of a drug efflux pump, as compared to a mock having added thereto dimethyl sulfoxide, the suppression of the cleavage of the APP was stronger than the suppression of the cleavage of the Notch at each of concentrations of 1 µM, 3 µM, and 10 µM (Fig. 7). Also in the yeast, APP-specific inhibition by begacestat was observed, and hence a result reinforcing the following fact was obtained: as compared to DAPT (Fig. 7) that suppressed the cleavage of each of the APP and the Notch, the Pf3FTX had an APP-specific cleavage-suppressing effect comparable to that of begacestat. While DAPT and begacestat are low-molecular weight compounds, the Pf3FTX-1, the Pf3FTX-2, and the Pf3FTX-4 each serving as a medium-molecular weight compound of a peptide may avoid side effects observed in the low-molecular weight compounds because the medium-molecular weight compounds are considered to have action sites different from the low-molecular weight compounds.

## Claims

1. A γ-secretase inhibitor comprising a three-finger toxin.

2. An Aβ-peptide production inhibiitor comprising a three-finger toxin.

3. A pharmaceutical for preventing or treating dementia comprising a three-finger toxin.

4. The γ-secretase inhibitor according to claim 1, the Aβ-peptide production inhibiitor according to claim 2, or the pharmaceutical according to claim 3, wherein the three-finger toxin is derived from *Protobothrops flavoviridis.*

5. The γ-secretase inhibitor according to claim 1, the Aβ-peptide production inhibiitor according to claim 2, or the pharmaceutical according to claim 3, wherein the three-finger toxin is formed of:
a polypeptide including an amino acid sequence set forth in SEQ ID NO: 1 or an amino acid sequence obtained by substituting, deleting, or adding one or a plurality of amino acids in the amino acid sequence set forth in SEQ ID NO: 1;
a polypeptide including an amino acid sequence set forth in SEQ ID NO: 2 or an amino acid sequence obtained by substituting, deleting, or adding one or a plurality of amino acids in the amino acid sequence set forth in SEQ ID NO: 2;
a polypeptide including an amino acid sequence set forth in SEQ ID NO: 3 or an amino acid sequence obtained by substituting, deleting, or adding one or a plurality of amino acids in the amino acid sequence set forth in SEQ ID NO: 3; or
a polypeptide including an amino acid sequence set forth in SEQ ID NO: 4 or an amino acid sequence obtained by substituting, deleting, or adding one or a plurality of amino acids in the amino acid sequence set forth in SEQ ID NO: 4:
SEQ ID NO: 1: 
MKTLLLILGVVAFVYLEPGYTRKCCIYPKSALFCREVQICPADEGMCFKMWNSTDRLMERTYYVRG
CAAACPNPVGDEEVYCCVTDNCNK.
SEQ ID NO: 2: 
MKTLLLILGVVAFVYLDSGFSLICNTCNRTVRIPLIPCDPVTCPEGLDQCYANRTGILRMKVERG
CTTNCTQTLTLMCCKRDMCN
SEQ ID NO: 3: 
MKTHLLALVMVAFLYLEPGYTLRCHSCVGYVCKEFEFCKPEQKQCIYLINRKKYSGCSRNCPSAG
ANELVKCCATDRCNLEIFCW
SEQ ID NO: 4:
